(19) Europäisches Patentamt
European Patent Office
Office européen des brevets

(11) Veröffentlichungsnummer: **0 277 612 B1**

(12) **EUROPÄISCHE PATENTSCHRIFT**

(45) Veröffentlichungstag der Patentschrift: **28.07.93**

(21) Anmeldenummer: **88101345.2**

(22) Anmeldetag: **30.01.88**

(51) Int. Cl.5: **C07D 405/04**, C07D 413/04, C07D 417/04, A61K 31/40, A61K 31/41, A61K 31/535, A61K 31/54, C07D 409/14, C07D 405/14

# (54) Substituierte 3,4-Dihydro-2H-benzopyrane, Verfahren zu ihrer Herstellung, ihre Verwendung sowie pharmazeutische Präparate auf Basis dieser Verbindungen.

(30) Priorität: **04.02.87 DE 3703227**

(43) Veröffentlichungstag der Anmeldung:
**10.08.88 Patentblatt 88/32**

(45) Bekanntmachung des Hinweises auf die Patenterteilung:
**28.07.93 Patentblatt 93/30**

(84) Benannte Vertragsstaaten:
**AT BE CH DE ES FR GB GR IT LI LU NL SE**

(56) Entgegenhaltungen:
**EP-A- 0 076 075**
**EP-A- 0 107 423**
**EP-A- 0 120 428**
**EP-A- 0 173 848**
**EP-A- 0 277 611**

**JOURNAL OF MEDICINAL CHEMISTRY, Band 29, 1986, American Chemical Society, Washington, DC, US; V.A. ASHWOOD et al.: "Synthesis and antihypertensive activity of 4-(cyclic amido)-2H-1-benzopyrans", Seiten 2194-2201**

(73) Patentinhaber: **HOECHST AKTIENGESELLSCHAFT**
**Postfach 80 03 20**
**W-6230 Frankfurt am Main 80(DE)**

(72) Erfinder: **Englert, Heinrich Christian, Dr.**
**Stormstrasse 13**
**W-6238 Hofheim am Taunus(DE)**
Erfinder: **Lang, Hans-Jochen, Dr.**
**Rüdesheimer Strasse 7**
**W-6238 Hofheim am Taunus(DE)**
Erfinder: **Mania, Dieter, Dr.**
**Berliner Ring 5**
**W-6233 Kelkheim (Taunus)(DE)**
Erfinder: **Schölkens, Bernward, Dr.**
**Hölderlinstrasse 62**
**W-6233 Kelkheim (Taunus)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 277 612 B1

Rank Xerox (UK) Business Services
(3.10/3.6/3.3.1)

## Beschreibung

Die Erfindung betrifft 3,4-Dihydro-2H-benzo[b]pyrane der Formel I

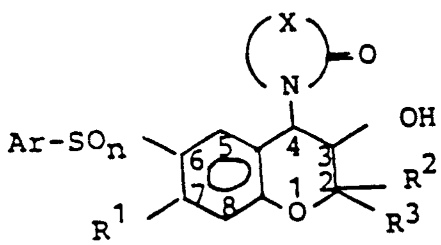

I

in welcher

- $R^1$ für H, OH, ($C_1$-$C_2$)-Alkoxy, ($C_1$-$C_2$)-Alkyl oder $NR^4R^5$ steht, wobei $R^4$ und $R^5$ gleich oder verschieden sind und für H, ($C_1$-$C_2$)Alkyl oder ($C_1$-$C_3$)-Alkylcarbonyl stehen,
- $R^2$,$R^3$ gleich oder verschieden sind und für Alkyl mit 1-4 C-Atomen stehen,
- Ar für Phenyl steht, das unsubstituiert oder durch 1 bis 3 gleiche oder verschiedene Reste ($C_1$-$C_2$)-Alkyl, ($C_1$-$C_2$)Alkoxy, Halogen, Trifluormethyl, CN, $NO_2$, CO-($C_1$-$C_2$)Alkyl $SO_m$-($C_1$-$C_2$)-Alkyl mit m = 1 oder 2 substituiert ist
- n für 1 oder 2 steht,
- X für eine Kette $(CH_2)_r$ steht, die durch ein Heteroatom O, S oder $NR^6$ unterbrochen sein kann, wobei $R^6$ H oder ($C_1$-$C_4$)-Alkyl bedeutet und r für die Zahlen 2, 3, 4 oder 5 steht.

Unter Halogen wird F, Cl, Br oder J, vorzugsweise F,Cl und Br verstanden.

Die C-Atome 3 und 4 des 3,4-Dihydro-2H-benzo[b]pyransystems (nachfolgend auch kurz "Chromansystem" genannt) der Formel I sind asymmetrisch substituiert. Gegenstand der Erfindung sind dabei nur solche Verbindungen, die an diesen Zentren entgegengesetzte Konfigurationen, also eine "trans"-orientierung" der Substituenten an diesen C-Atomen aufweisen. Enthält einer der Substituenten $R^1$, $ArSO_n$, $R^2$ und/oder $R^3$ Asymmetriezentren oder, falls $R^2$ und $R^3$ ungleich sind (und somit ein asymmetrisches Kohlenstoffatom erzeugen), sind Verbindungen mit sowohl S- als auch R-konfigurierten Zentren Gegenstand der Erfindung.

Die Verbindungen können als optische Isomere, als Diastereoisomere, als Racemate oder als Gemische derselben vorliegen.

Bevorzugt sind Verbindungen der Formel I, bei denen $R^1$ bis $R^3$, $ArSO_n$ die oben erwähnten Bedeutungen haben, X jedoch für eine Kette $(CH_2)_r$ steht mit r = 3 oder 4.

Ganz besonders bevorzugt sind solche Verbindungen, bei denen $R^1$ bis $R^3$ die oben erwähnten Bedeutungen haben, Ar für Phenyl steht, das unsubstituiert oder wie oben definiert substituiert ist, n für 2 steht und X für eine Kette $(CH_2)_r$ mit r = 3 oder 4.

Insbesondere bevorzugt sind solche Verbindungen, bei denen $R^1$ H oder ($C_1$-$C_2$)-Alkoxy bedeutet, $R^2$ und $R^3$ die oben erwähnte Definition haben, Ar für Phenyl steht, das unsubstituiert oder durch ($C_1$-$C_2$)-Alkyl, Cyano, ($C_1$-$C_2$)Alkoxy oder Halogen einfach substituiert ist, n für 2 steht und X für eine Kette $(CH_2)_r$ mit r = 3 oder 4.

In J.Med.Chem. 1986, 29, 2194-2201 werden Verbindungen beschrieben, die den erfindungsgemäßen Verbindungen strukturell am nächsten stehen. Sie sind dort unter folgenden allgemeinen Formel zusammengefaßt:

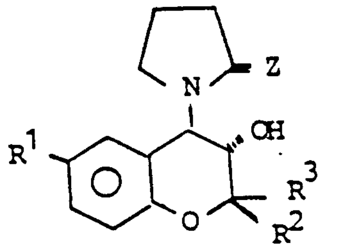

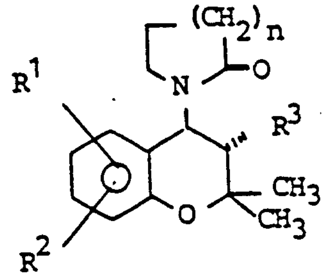

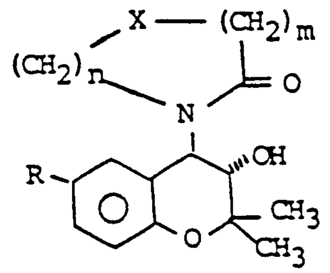

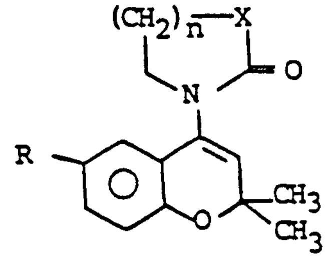

mit $R^1$, $R^2$, $R^3$, Z, n, m, R in den dort aufgeführten Bedeutungen. Ein großer Teil dieser Verbindungen war auch Gegenstand verschiedener Patentanmeldungen, zu nennen sind hierbei EP 0 107 423, EP 0 120 427, EP 0 076 075 EP 0 120 428.

Insbesondere sind in der oben zitierten Literaturstelle solche Verbindungen als besonders wirksam beschrieben worden, die in der 6-Position des 3,4-Dihydro-2H-benzopyransystems eine CN oder eine $NO_2$-Gruppe tragen, wobei insbesondere dem (±)-6-Cyano-3,4-dihydro-2,2-dimethyl-trans-4-(2-oxo-1-pyrrolidinyl)-2H-benzo[b]pyran-3-ol eine besondere Bedeutung zukommt. Verbindungen, die in der oben erwähnten 6-Position Sulfonylalkylreste oder Sulfoxyalkylreste tragen, sind in den Ansprüchen der oben genannten Patentanmeldungen enthalten, wurden aber bislang nicht realisiert und daher auch nicht auf ihre Wirksamkeit hin untersucht.

In einer weiteren Patentschrift (EP 0 173 848) wurde die Anwendung dieser Verbindungen auch bei anderen cardiovaskulären Erkrankungen als sinnvoll beschrieben Insbesondere bei der Herzinsuffizienz oder bei Angina pectoris kann eine relaxierende Wirkung auf das Gefäßsystem von großem therapeutischen Nutzen sein. Die in EP 0 173 848 aufgeführten Experimente an isolierten Gefäßen weisen auf einen derartigen Effekt der Verbindungen hin.

Mit der erfindungsgemäßen Verbindungen I wurde nun eine neue Substanzklasse mit günstigem Einfluß auf das cardiovaskuläre System gefunden. Überraschenderweise führte die Kombination eines Arylrestes Ar mit einer Sulfonyl-bzw. Sulfoxygruppe als Substituent für die 6-Position des Chromansystems zu verbesserten Wirkeigenschaften. Die beobachtete Überlegenheit kann die Potency bezüglich der Blutdrucksenkung und/oder der Relaxation bestimmter Gefäßsysteme wie etwa der Aorta thoracica oder des Coronarsystems betreffen. Darüber hinaus beobachtet man am Modell des Langendorff-Herzens in vielen Fällen einen weitaus länger anhaltenden Einfluß auf die Coronardurchströmung als dies bei den oben genannten bekannten Verbindungen der Fall ist. Hierdurch sind Verbindungen I wertvolle Therapeutika, die sich für die Behandlung des erhöhten Blutdrucks, für die Therapie der Angina pectoris sowie der Herzinsuffizienz eignen. Da diese Krankheiten auch miteinander kombiniert auftreten können, kommt den Verbindungen I zusätzliche Bedeutung zu.

Die Erfindung betrifft weiterhin ein Verfahren zur Herstellung der Verbindungen I, dadurch gekennzeichnet, daß man

a) Verbindungen der Formel II

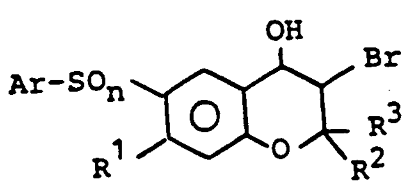

II,

in denen $R^1$ bis $R^3$ und $ArSO_n$ wie oben definiert sind umsetzt mit Lactamen der Formel III

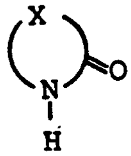

III,

b) Verbindungen der Formel IV

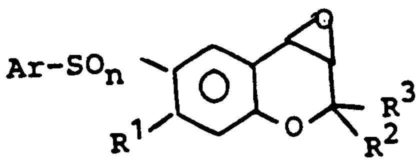

IV,

in denen $R^1$ bis $R^3$ und $ArSO_n$ wie oben definiert sind, umsetzt mit den Lactamen der Formel III

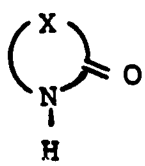

c) Verbindungen der Formel V

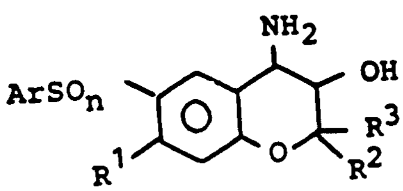

V,

in denen $R^1$ bis $R^3$ und $ArSO_n$ wie oben definiert sind, acyliert zu den Verbindungen VI

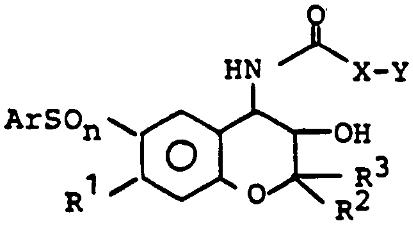

VI,

in denen Y eine Fluchtgruppe, wie etwa Chlor oder Brom, ist und $R^1$ bis $R^3$ und $ArSO_n$ wie oben definiert sind, und diese zu den Verbindungen I cyclisiert

d) Verbindungen der Formel VII

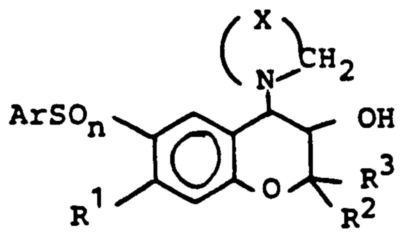

VII,

in denen $R^1$ bis $R^3$ und $ArSO_n$ wie oben definiert sind, zu den Verbindungen I oxidiert.

Werden die Verbindungen I nach den Methoden a) oder b) hergestellt, so geschieht dies dadurch, daß man die Verbindungen II oder IV in einem geeigneten Lösemittel, vorzugsweise in dipolar aprotischen Lösemitteln wie etwa Dimethylsulfoxid oder THF umsetzt mit den Lactamen III, vorzugsweise unter Einwirkung von Basen, wie ewa Natriumhydrid, K-tert.butylat oder ähnlichen, für Lactam-N-Alkylierungen bekanntermaßen geeigneten Basen. Die Reaktionstemperatur ist dabei im weiten Bereich variierbar; vorzugsweise wird zwischen 0° und Zimmertemperatur gearbeitet oder bei Temperaturen, die leicht oberhalb der Zimmertemperatur liegen können.

Lactame der Formel III sind in vielen Fällen bekannt, oder können leicht nach literaturbekannten Methoden hergestellt werden. Verbindungen II oder IV sind neu. Sie können beispielsweise nach folgendem Syntheseweg hergestellt werden:

Verbindungen der Formel VIII

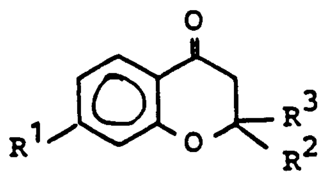

VIII,

in denen $R^1$, $R^2$ und $R^3$ wie oben definiert sind, werden mit Säurechloriden $Ar\text{-}SO_n\text{-}Cl$ in an sich bekannter Weise nach Art der Friedel-Crafts Acylierung umgesetzt zu Verbindungen der Formel IX

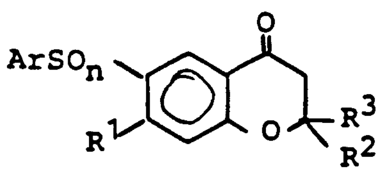

IX,

in denen $R^1$, $R^2$, $R^3$ und Ar und n wie oben definiert sind. Diese werden durch Reduktionen unter Standardbedingungen etwa durch $NaBH_4$ in Methanol umgesetzt zu den Verbindungen X

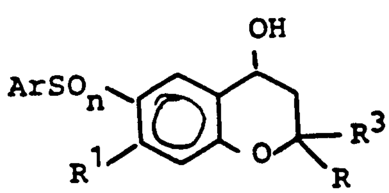

X

und anschließend einer Wasserabspaltung, etwa durch Pyridin/Phosphoroxychlorid, unterworfen, wobei Verbindungen der Formel XI entstehen:

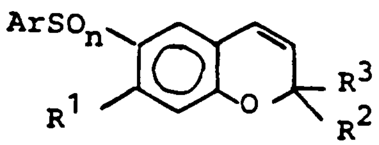

XI

Verbindung XI lassen sich nun leicht nach Standardmethoden in die Epoxide IV oder die Bromhydrine II umwandeln.

Steht bei dieser Reaktionssequenz $R^1$ in der Bedeutung von $NH_2$ oder OH, so sind gegebenenfalls Schutzgruppen wie etwa die Dimethylaminomethylengruppe für $NH_2$ oder die Acetyl- oder Methylgrupe für die OH-Gruppe notwendig. Diese werden auf geeigneten Stufen, vorzugsweise nach Durchführung der in Verfahren a) oder b) beschriebenen Umsetzungen durch gängige Methoden wieder abgespaltet.

Chromene der Formel XI werden in einigen Fällen in an sich bekannter Weise durch thermisch induzierte Cyclisierung der entsprechenden Propargylether XII

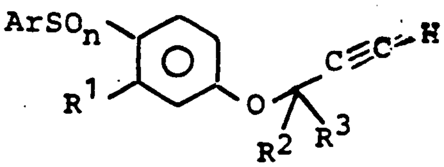

XII

hergestellt. Diese wiederum lassen sich in an sich bekannter Weise aus den Phenolen XIII und den Propargylchloriden XIV herstellen.

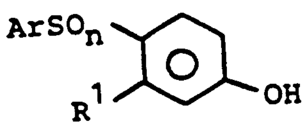

XIII

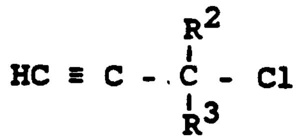

XIV

Die Verfahren c) und d) können dann besonders günstig angewandt werden, wenn enantiomerenreine Endprodukte I erwünscht sind. Verbindungen V und VII sind im Gegensatz zu Verbindungen I basisch und damit zur Salzbildung mit organischen Säuren befähigt. Durch Kristallisation mit einer geeigneten optimisch einheitlichen Säure wie etwa (+) Mandelsäure oder (+)-Milchsäure können sie in an sich bekannter Weise enantiomerenrein erhalten werden und in enantiomerenreine Endprodukte I nach der Verfahren c) und d) umgewandelt werden.

Enantiomerenreine Endprodukte I können aber auch aus racemischen Endprodukten I durch gängige Racematspaltungsmethoden wie etwa die chromatographische Trennung unter Verwendung von chiralen Phasen oder die Derivatisierung der racemischen Produkte mit optisch einheitlichen Säurederivaten (Esterbildung über die 3-Hydroxygruppe des Chromansystems) oder mit optisch einheitlichen Isocyanaten (Carbamatbildung über die 3-Hydroxygruppe) aufgespalten werden. Die dabei erhaltenen diastereoisomeren Isocyanate oder Ester lassen sich durch gängige Methoden (Kristallisation oder Chromatographie) trennen und unter Abspaltung der optisch aktiven Hilfsgruppe an der 3-OH-Funktion in die optisch einheitlichen Endverbindungen I umwandeln. Als besonders vorteilhaft hat sich hierbei die Trennung der diastereomeren 3-Menthoxyacetate erwiesen.

Die erfindungsgemäßen Verbindungen I können wie bereits erwähnt als Antihypertensiva, als Coronartherapeutika oder als Mittel zur Behandlung der Herzinsuffizienz eingesetzt werden. Sie können dabei sowohl in der Human als auch in der Veterinärmedizin zur Anwendung gelangen. Sie werden in Dosierungen von mindestens 0,002 mg/kg/Tag, vorzugsweise mindestens 0,01 mg/kg/Tag und insbesondere mindestens 0,1 mg/kg/Tag bis maximal 20 mg/kg/Tag, vorzugsweise bis 10 mg/kg/Tag und insbesondere bis 4 mg/kg/Tag in Kapseln, Dragees, Tabletten, Puder, Zäpfchen oder Lösungen mit Zusätzen oder ohne

Zusätze von galenischen Hilfsstoffen enteral, z.B. oral oder parenteral (wie etwa durch Injektion in das Gefäßsystem, z.B. intravenös) verabfolgt. Diese Angaben beziehen sich auf einen erwachsenen Menschen von 75 kg Körpergewicht. Die Verbindungen I eignen sich zur Behandlung von cardiovaskulären Erkrankungen, wie der Hhpertonie, der Angina pectoris oder der Herzinsuffizienz allein oder in Kombination mit anderen Herz-Kreislauf-aktiven Arzneimitteln,wie Diuretika, Ca-Antagonisten ACE-Hemmern, oder $\beta$-Sympathikolytica .

Erfindungsgemäß können beispielsweise die in der folgenden Tabelle zusammengestellten Verbindungen der Formel I erhalten werden.

1) 2,2-Dimethyl-3,4-dihydro-7-methoxy-6-(p-chlorphenylsulfonyl)-trans-4-(2-oxo-1-pyrrolidinyl)-2H-benzo-[b]pyran-3-ol,
2) 2,2-Dimethyl-3,4-dihydro-6-(p-chlorphenylsulfonyl)-trans-4-(2-oxo-1-piperidinyl)-2H-benzo[b]pyran-3-ol,
3) 2,2-Dimethyl-3,4-dihydro-6-(p-nitrophenylsulfonyl)-trans-4(2-oxo-1-pyrrolidinyl)-2H-benzo[b]pyran-3-ol,
4) 2,2-Dimethyl-3,4-dihydro-6-(p-cyanophenylsulfonyl)-trans-4-(2-oxo-1-pyrrolidinyl)-2H-benzo[b]pyran-3-ol,
5) 2,2-Dimethyl-3,4-dihydro-6-(p-methoxyphenylsulfonyl)-trans-4-(2-oxo-1-pyrrolidinyl)-2H-benzo[b]pyran-3-ol,
6. 2,2-Dimethyl-3,4-dihydro-6-(p-trifluormethylphenylsulfonyl)-trans-4-(2-oxo-1-pyrrolidinyl)-2H-benzo[b]-pyran-3-ol,
7. 2,2-Dimethyl-3,4-dihydro-6-(p-methylsulfonylphenylsulfonyl)-trans-4-(2-oxo-1-pyrrolidinyl)-2H-benzo[b]-pyran-3-ol,
8) 2,2-Dimethyl-3,4-dihydro-6-(p-acetylphenylsulfonyl)-trans-4-(2-oxo-1-pyrrolidinyl)-2H-benzo[b]pyran-3-ol,
9. 2,2-Dimethyl-3,4-dihydro-7-methylamino-6-phenylsulfonyl-trans-2(2-oxo-1-pyrrolidinyl)-2H-benzo[b]-pyran-3-ol,
10) 2,2-Dimethyl-3,4-dihydro-7-fluor-6-phenylsulfonyl-trans-(2-oxo-1-pyrrolidinyl)-2H-benzo[b]pyran-3-ol,
11) 2,2-Diethyl-3,4-dihydro-7-fluor-6-phenylsulfonyl-trans-(2-oxo-1-pyrrolidinyl)-2H-benzo[b]pyran-3-ol,
12) 2,2-Dimethyl-7-chlor-3,4-dihydro-6-phenylsulfonyl-trans-(2-oxo-1-pyrrolidinyl)-2H-benzo[b pyran-3-ol,
13) 2,2-Dimethyl-3,4-dihydro-6-(4-chlor-3-methylphenylsulfonyl)-trans-(2-oxo-1-pyrrolidinyl)-2H-benzo[b]-pyran-3-ol,
14) 2,2-Dimethyl-3,4-dihydro-trans-4-(4-methyl-2-oxo-1-piperazinyl)-6-phenylsulfonyl-2H-benzo[b]pyran-3-ol,
15) 2,2-Dimethyl-3,4-dihydro-6-phenylsulfonyl-trans-4-(2-oxo-1-morpholinyl)-2H-benzo[b]pyran-3-ol,
16) 2,2-Dimethyl-3,4-dihydro-6-phenylsulfonyl-trans-4-(5-oxo-3-oxazolidinyl)-2H-benzo[b]pyran-3-ol.
17) 3,4-Dihydro-2,2-dimethyl-6-(p-fluorphenylsulfonyl)-trans-4-(2-oxo-1-pyrrolidinyl)-2H-benzo[b]pyran-3-ol,
18) 3,4-Dihydro-2,2-dimethyl-6-(o-fluorphenylsulfonyl)-trans-4-(2-oxo-1-pyrrolidinyl)-2H-benzo[b]pyran-3-ol,
19) 3,4-Dihydro-2,2-dimethyl-6-(3-pyridylsulfonyl)-trans-4-(2-oxo-1-pyrrolidinyl)-2H-benzo[b]pyran-3-ol,
20) 3,4-Dihydro-2,2-dimethyl-6-(2-pyrimidinylsulfonyl)-trans-4-(2-oxo-1-pyrrolidinyl)-2H-benzo[b]pyran-3-ol,
21) 3,4-Dihydro-2,2-dimethyl-6-(2-furylsulfonyl)-trans-4-(2-oxo-1-pyrrolidinyl)-2H-benzo[b]pyran-3-ol.

Beispiel 1

3,4-Dihydro-2,2-dimethyl-7-methoxy-6-(p-tolylsulfonyl)-trans-4-(2-oxo-1-pyrrolidinyl)-2H-benzo[b]pyran-3-ol

4,3 g (0,0097 Mol) 3-Brom-3,4-Dihydro-2,2-dimethyl-7-methoxy-6-(p-tolylsulfonyl)-2H-benzo[b]pyran-4-ol werden in 28 ml Dimethylsulfoxid gelöst, mit 3,5 ml 2-Pyrrolidinon (0,0465 Mol) und 0,78 g Natriumhydrid (80 %ige Suspension in Öl) (0,0325 Mol) versetzt und 3 Stunden bei 40 °C gerührt. Man läßt über Nacht stehen, gießt den Ansatz auf Eiswasser, und saugt ab. Der Niederschlag wird aus Isopropanol umkristallisiert. Weiße Kristalle vom Schmp.: 263 - 65 °C.

Herstellung der Ausgangsverbindung:

3-Brom-3,4-dihydro-2,2-dimethyl-7-methoxy-6-(p-tolylsulfonyl)-2H-benzo[b]pyran-4-ol erhält man aus 2,2-Dimethyl-7-methoxy-6-(p-tolylsulfonyl)-2H-chromen und N-Bromsuccinimid in einem 9:1 Gemisch aus Dimethylsulfoxid/$H_2O$. Schmp.: 200-201 °C
2,2-Dimethyl-7-methoxy-6-(p-tolylsulfonyl)-2H-chromen erhält man aus 2,2-Dimethyl-7-methoxy-6-(p-tolylsulfonyl)-chroman-4-ol mit Phosphoroxychlorid/Pyridin in Benzol.
Schmp.: 132 - 33 °C
2,2-Dimethyl-7-methoxy-6-(p-tolylsulfonyl)-chroman-4-ol erhält man aus 2,2-Dimethyl-7-methoxy-6-(p-tolylsulfonyl)chroman-4-on mit $NaBH_4$ in Ethanol.
Schmp.: 196 - 97 °C
2,2-Dimethyl-7-methoxy-6-(p-tolylsulfonyl)chroman-4-on erhält man aus 2,2-Dimethyl-7-methoxy-chroman-4-on und p-Toluolsulfonsäurechlorid in Gegenwart von Aluminiumchlorid in Methylenchlorid.
Schmp.: 221 - 23 °C.

Beispiel 2

3,4-Dihydro-2,2-dimethyl-7-methoxy-6-(p-tolylsulfonyl)-trans-4-(2-oxo-1-piperidinyl)-2H-benzo[b]pyran-3-ol

5 g (0,011 Mol) 3-Brom-3,4-dihydro-2,2-dimethyl-7-methoxy-6-(p-tolylsulfonyl)-2H-benzo[b]pyran-4-ol werden in 32 ml Dimethylsulfoxid gelöst und mit 4,9 g Valerolactam (0,0526 Mol) sowie 0.8 g (0,033 Mol)-NaH, 80 %ige Suspension in Öl, versetzt und 5 Stunden bei 40 °C gerührt. Man gießt den Ansatz auf Eiswasser und saugt ab. Der Rückstand wird mehrfach mit Methanol ausgekocht.
Weiße Kristalle vom Schmp.: 261 - 63 °C

Beispiel 3

3,4-Dihydro-2,2-dimethyl-7-methoxy-6-phenylsulfonyl-trans-4-(2-oxo-1-pyrrolidinyl)-2H-benzo[b]pyran-3-ol

Die Verbindung wird analog Beispiel 1 aus 3-Brom-3,4-dihydro-2,2-dimethyl-7-methoxy-6-phenylsulfonyl-2H-benzo[b]pyran-4-ol hergestellt.
Weiße Kristalle vom Schmp.: 227 - 29 °C

Trennung der Antipoden, Beispiel 3a

1,075 g (0,0025 Mol) (±)-3,4-Dihydro-2,2-dimethyl-7-methoxy-6-phenylsulfonyl-trans-4-(2-oxo-1-pyrrolidinyl)-2H-benzo[b]pyran-3-ol werden in 5 ml 1,2-Dichlorbenzol gelöst, mit 0,9 g S(-)-1-Phenylethylisocyanat versetzt und ca 12 h bei 140 °C gerührt. Der komplette Ansatz wird anschließend an Kieselgel mit dem Laufmittelsystem Toluol/Essigester 1:1 chromatographiert. Das langsamer laufende diastereomere Carbamat kann angereichert werden und durch Kristallisation aus Toluol rein erhalten werden (Schmp. 243-245 °C). Durch Verseifung mit NaOH in EtOH bei 80 °C erhält man das (+)-3,4-Dihydro-2,2-dimethyl-7-methoxy-6-phenylsulfonyl-trans-4-(2-oxo-1-pyrrolidinyl)-2H-benzo[b]pyran-3-ol mit dem Schmp.: 209-211 °C und $[\alpha]_D$ = + 109° (C = 0,28; $CHCl_3$)

Herstellung des Ausgangsmaterials:

3-Brom-3,4-dihydro-2,2-dimethyl-7-methoxy-6-phenylsulfonyl-2H-benzo[b]pyran-4-ol erhält man aus 2,2-Dimethyl-7-methoxy-6-phenylsulfonyl-2H-chromen und N-Bromsuccinimid in einem 9:1 Dimethylsulfoxid/$H_2O$
Gemisch. Schmp.: 202 - 203 °C.
2,2-Dimethyl-7-methoxy-6-phenylsulfonyl-2H-chromen erhält man aus 2,2-Dimethyl-4-hydroxy-7-methoxy-6-phenylsulfonylchromen mit Pyridin/Phosphoroxychlorid in Benzol.
Schmp: 140 - 41 °C.
2,2-Dimethyl-4-hydroxy-7-methoxy-6-phenylsulfonylchroman erhält man aus 2,2-Dimethyl-7-methoxy-6-phenylsulfonylchroman-4-on mit Natriumborhydrid in Methanol.
Schmp.: 146 - 147 °C.
2,2-Dimethyl-7-methoxy-6-phenylsulfonylchroman-4-on erhält man aus Phenylsulfonylchlorid, 2,2-Dimethyl-7-methoxychroman-4-on und Aluminiumchlorid in Methylenchlorid.
Schmp.: 223 - 25 °C

Beispiel 4

3,4-Dihydro-2,2-dimethyl-6-(4 -methylphenylsulfonyl)-trans-4-(2 -oxo-1-pyrrolidinyl)-2H-benzo[b]pyran-3-ol

Zu 8,2 g (0,02 Mol) 3-Brom-3,4-dihydro-2,2-dimethyl-6-(4′-methylphenylsulfonyl)-2H-benzo[b[pyran-4-ol in 30 ml Dimethylsulfoxid werden 0,75 g (0,025 Mol) 80 %iges NaH eingetragen. Nach einstündigem Rühren bei 20° werden weitsdere 0,75 g (0,025 Mol) 80 % NaH und 1,9 ml (0,025 Mol) 2-Pyrrolidon zugefügt und 45 Minuten bei 40° und 6 Stunden bei 20° gerührt. Nach Eintragen in Eiswasser wird der Niederschlag abgesaugt, getrocknet und mehrfach aus Methanol umkristallisiert.
Kirstalle von Schmp.: 242 - 243°C.

Herstellung des Ausgangsmaterials

3-Brom-3,4-dihydro-2,2-dimethyl-6-(4-methylphenylsulfonyl)-2H-benzo[b]pyran-4-ol

Zu 12,6 g (0,04 Mol) 2,2-Dimethyl-6-(4′-methyl)-phenylsulfonyl-chromen in einer Lösung aus 70 ml Dimethylsulfoxid und 1,4 ml Wasser werden unter Kühlung (Isopropanol/Trockeneis) bei ungefähr 15°C 14,2 g (0,08 Mol) frisch umkristallisiertes N-Bromsuccinimid eingetragen. Die Temperatur steigt vorübergehend auf 27°. Es wird auf 20°C abgekühlt und nach einstündigem Rühren in Eis/Essigester eingetragen. Die Essigesterphase wird mehrfach mit Wasser gewaschen und über $Na_2SO_4$ getrocknet. Beim Einengen kristallisiert das Bromhydrin-Derivat.
Kristalle vom Schmp.: 141 - 142°C.

Beispiel 5

3,4-Dihydro-2,2-dimethyl-6-phenylsulfonyl-trans-4-(2-oxo-1-pyrrolidinyl)-2H-benzo[b]pyran-3-ol

Zu einer Suspension von 0,6 g (0,02 Mol) 80 %igem NaH in 10 ml DMSO wird eine Lösung aus 6,3 g (0,02 Mol) 3,4-Dihydro-2,2-dimethyl-3,4-epoxi-6-phenylsulfonyl-2H-benzo[b]pyran in 20 ml DMSO bei 20° zugetropft. Anschließend werden 2,3 ml (0,03 Mol) 2-Pyrrolidinon zugefügt und 1 Stunde bei 45° gerührt. Nach Stehen über nacht bei 20° wird in Eiswasser eingetragen. Der Niederschlag wird abgesaugt, neutral gewaschen, getrocknet und an Kieselgel mit Methylenchlorid/Methanol 19:1 chromatographiert. 30 ml-Fraktionen werden aufgefangen. Die Fraktionen 12-25 werden eingeengt und der Rückstand wird aus Acetonitril umkristallisiert.
Schmp.: 201-202°

Herstellung des Ausgangsmaterials:

3,4-Dihydro-2,2-dimethyl-3,4-epoxi-6-phenylsulfonyl-2H-benzo[b]pyran erhält man aus 3-Brom-3,4-dihydro-2,2-dimethyl-6-phenylsulfonyl-2H-benzo[b]pyran-4-ol mit NaH in DMSO.
Schmp.: 103-105°

3-Brom-3,4-dihydro-2,2-dimethyl-6-phenylsulfonyl-2H-benzo[b]pyran-4-ol erhält man aus 2,2-Dimethyl-6-phenylsulfonyl-2H-chromen und N-Bromsuccinimid in einem 9:1 Dimethylsulfoxid/$H_2O$ Gemisch
Schmp.: 126°

Das 2,2-Dimethyl-6-phenylsulfonyl-2H-chromen mit dem Schmp.: 70-71° wurde nach bekannten Methoden aus 6-Phenylsulfonylphenyl-1,1 -dimethylpropargylether hergestellt. Diesen Ether erhielt man in ebenfalls bekannter Weise aus 6-Phenylsulfonylphenol und 3-Methyl-3-chlorbutin.
(+)-3,4-Dihydro-2,2-dimethyl-6-phenylsulfonyl-4-(2-oxo-1-pyrrolidinyl)-2H-benzo[b]pyran-3-ol (Beispiel 5a)
(±)-3,4-Dihydro-2,2-dimethyl-6-phenylsulfonyl-4-(2-oxo-1-pyrrolidinyl)-2H-benzo[b]pyran-3-ol wird nach Standardmethoden mit (-)Menthoxyessigsäurechlorid verestert. Die diastereomeren Ester werden auf einer Kieselgelsäule mit Methylenchlorid/Essigester (9:1) getrennt und mit alkoholischer Natriumethylatlösung unter Rühren bei 20° verseift. Nach Verdünnen mit kaltem Wasser wird der Niederschlag abgesaugt und neutral gewaschen und mit Äther verrieben.
(+)-3,4-Dihydro-2,2-dimethyl-6-phenylsulfonyl-4-(2 -oxo-1-pyrrolidinyl)-2H-benzo[b]pyran-3-ol
Schmp.: 122-123° $[\alpha]_D$ = + 39,5° (c= 1 , Ethanol)

Beispiel 6

6-(4-Chlorphenylsulfonyl)-3,4-dihydro-2,2-dimethyl-7-methoxy-trans-4-(2-oxo-1-pyrrolidinyl)-2H-benzo[b]-pyran-3-ol

Die Verbindung wird analog Beispiel 1 aus 3-Brom-6-(4-Chlorphenylsulfonyl)-3,4-dihydro-2,2-dimethyl-7-methoxy-2H-benzo[b]pyran-4-ol hergestellt.
Weiße Kristalle mit Schmp.: 260-262 °C

Herstellung der Ausgangsverbindungen:

Analog Beispiel 1:
3-Brom-6-(4-Chlorphenylsulfonyl)-3,4-dihydro-2,2-dimethyl-7-methoxy-2H-benzo[b]pyran-4-ol mit Schmp.: 175-177 °C
6-(4-Chlorphenylsulfonyl)-2,2-dimethyl-7-methoxy-chromen mit Schmp.: 142-143 °C

Beispiel 7

6-(4-Bromphenylsulfonyl)-3,4-dihydro-2,2-dimethyl-7-methoxy-trans-4-(2-oxo-1-pyrrolidinyl)-2H-benzo[b]-pyran-3-ol

Analog Beispiel 1 aus 3-Brom-6-(4-Bromphenylsulfonyl)-3,4-dihydro-2,2-dimethyl-7-methoxy-2H-benzo-[b]pyran-4-ol.
Weiße Kristalle vom Schmp.: 281-282 °C.

Beispiel 8

3,4-Dihydro-2,2-dimethyl-7-methoxy-6-)4-methoxyphenylsulfonyl)-trans-4-(2-oxo-1-pyrrolidinyl)-2H-benzo[b]-pyran-3-ol

Die Verbindung wird analog Beispiel 1 aus 3-Brom-3,4-dihydro-2,2-dimethyl-7-methoxy-6-(4-methoxy-phenylsulfonyl)-2H-benzo[b]pyran-4-ol hergestellt und hat einen Schmp. von 286-287 °C.

Beispiel 9

3,4-Dihydro-2,2-dimethyl-7-methoxy-6-(2-thienylsulfonyl)-trans-4-(2-oxo-1-pyrrolidinyl)-2H-benzo[b]pyran-3-ol

Analog Beispiel 1 aus 3-Brom-3,4-dihydro-2,2-dimethyl-7-methoxy-6-(2-thienylsulfonyl)-2H-benzo[b]-pyran-4-ol,
Schmp.: 135-136 °C.

Beispiel 10

3,4-Dihydro-2,2-dimethyl-7-ethoxy-6-phenylsulfonyl-trans-4-(2-oxo-1-pyrrolidinyl)-2H-benzo[b]pyran-3-ol

Analog Beispiel 1 aus 3-Brom-3,4-dihydro-2,2-dimethyl-7-ethoxy-6-phenylsulfonyl-2H-benzo[b]pyran-4-ol,
Schmp.: 197-198 °C.

Beispiel 11

3,4-Dihydro-2,2-dimethyl-7-methoxy-6-phenylsulfonyl-trans-4-(2-oxo-1-piperidinyl)-2H-benzo[b]pyran-3-ol

Analog Beispiel 2 aus 3-Brom-3,4-dihydro-2,2-dimethyl-7-methoxy-6-phenylsulfonyl-2H-benzo[b]pyran-4-ol,
Weiße Kristalle von Schmp.: 157-158 °C.

Beispiel 12

6-(4-Cyanphenylsulfonyl)-3,4-dihydro-2,2-dimethyl-trans-4-(2-oxo-1-pyrrolidinyl)-2H-benzo[b]pyran-3-ol

Analog Beispiel 1 aus 3-Brom-6-(4-cyanphenylsulfonyl)-3,4-dihydro-2,2-dimethyl-2H-benzo[b]pyran-4-ol, Weiße Kristalle vom Schmp.: 234-235 °C.

Herstellung des Ausgangsmaterials:

3-Brom-6-(4-cyanphenylsulfonyl)-3,4-dihydro-2,2-dimethyl-2H-benzo[b]pyran-4-ol erhält man wie unter Beispiel 3 beschrieben aus 6-(4-Cyanphenylsulfonyl)-2,2-dimethyl-3 -chromen.
Schmp.: 157-158 °C.

Beispiel 13

3,4-Dihydro-2,2-dimethyl-6-(2-methoxyphenylsulfonyl)-trans-4-(2-oxo-1-pyrrolidinyl)-2H-benzo[b]pyran-3-ol

Analog Beispiel 1 aus 3-Brom-3,4-dihydro-2,2-dimethyl-6-(2-methoxyphenylsulfonyl)-2H-benzo[b]pyan-3-ol.
Weiße Kristalle vom Schmp.: 196-198 °C.

Beispiel 14

3,4-Dihydro-2,2-dimethyl-6-(2-methylphenylsulfonyl)-trans-4-(2-oxo-1-pyrrolidinyl)-2H-benzo[b]pyran-3-ol

Analog Beispiel 1. Weiße Kristalle vom Schmp.: 214-216 °C.

Beispiel 15

3,4-Dihydro-2,2-dimethyl-6-(2-chlorphenylsulfonyl)-trans-4-(2-oxo-1-pyrrolidinyl)-2H-benzo[b]pyran-3-ol

Analog Beispiel 1. Weiße Kristalle vom Schmp.: 85-87 °C

Beispiel 16

Herstellung von 3,4-Dihydro-2,2-dimethyl-6-phenylsulfonyl-trans-4-(2-oxo-1-pyrrolidinyl)-2H-benzo[b]pyran-3-ol (Verbindung von Beispiel 5 nach Verfahrensvariante c)

Eine Lösung von 3-Brom-3,4-dihydro-2,2-dimethyl-6-phenylsulfonyl 2H-benzo[b]pyran-4-ol in Ethanol wird 8 Stunden bei 50° im Autoklaven bei einem Druck von 8 bar $NH_3$ geschüttelt. Nach dem Abkühlen wird bis zur Trockene eingeengt und aus Essigester umkristallisiert. Man erhält das 4-Amino-3,4-dihydro-2,2-dimethyl-6-phenylsulfonyl-2H-benzo[b]pyran-3-ol vom Schmp.: 160-163 °C, das sofort einer Acetylierung mit 5-Chlorbuttersäurechlorid unterworfen wird. Hierzu wird die Substanz sowie das Säurechlorid in $CH_2Cl_2$ und im Zweiphasengemisch mit 2N Natronlauge 24 Stunden bei Zimmertemperatur gerührt. Nach üblicher Aufarbeitung erhält man das 4-(5-Chlorbutyrylamino)-3,4-dihydro-2,2-dimethyl-6-phenylsulfonyl-2H-benzo[b]-pyran-4-ol vom Schmp.: 155-157 °C. Die Cyclisierung zur Titelverbindung erfolgt durch Lösung der Substanz in Tetrahydrofuran, Zugabe einer stöchiometrischen Menge 80 %igem NaH-Suspension inÖl undRühren des Gemisches bei Zimmertemperatur über 24 Stunden. Das Endprodukt ist mit dem nach Verfahren b) gewonnenen Produkt identisch. Schmp.: 200-201 °C. Wird das 4-Amino-3,4-dihydro-2,2-dimethyl-6-phenylsulfonyl-2H-benzo[b]pyran-3-ol einer Racematspaltung unterworfen, so kann aus seinem (+)-Enantiomer das reine (+)-Enantiomer aus Beispiel 5a mit den dort angegebenen Daten erhalten werden.

Beispiel 17

3,4-Dihydro-2,2-dimethyl-6-phenylsulfoxy-trans-4-(2-oxo-1-pyrrolidinyl)-2H-benz[b]pyran-3-ol

Analog Beispiel 1. Schmp.: 211 - 212 °C

Pharmakologische Daten

Koronardurchströmung am Langendorff-Herz

a) Methode

Meerschweinchen beiderlei Geschlechts werden durch einen Schlag auf den Nacken getötet. Die Herzen werden rasch entnommen und nach der Methode von Langendorff (Langendorff, Pflügers Arch. Ges. Physiol. 190, 280 (1895)) mit einer Ringerlösung perfundiert. Die Lösung enthalt 154 mMol NaCl, 5,6 mMol KCl, 1,9 mMol $CaCl_2$, 2,4 mMol $NaHCO_3$ und 5 mMol Glucose und wurde mit Sauerstoff begast. Ihre Temperatur betrug 37 °C. Die Testsubstanzen ((0,1 %ige Lösung in Propandiol) wurden als Bolusinjektion in den Zuführungsschlauch der einströmenden Ringerlösung zugesetzt. Die Wirkung, d. h. Veränderungen des Koronarflusses, wurde mit Hilfe eines Tropfenzählers bestimmt und mit Hilfe eines Heilige Multiskriptors (Hellige GmbH, Freiburg, Bundesrepulik Deutschland) registriert. Jede Verbindung wurde in 3 verschiedenen Dosierungen an je 6 verschiedenen Herzpräparationen getestet. Aus diesen Daten wurden $ED_{50}$-Werte berechnet (Probit-Analyse und lineare Regression) als diejenigen Dosen, bei der eine 50 %ige Zunahme des Koronarflusses zu erwarten ist. Die Dauer des erhöhten Koronardurchflusses wurde an je 6 verschiedenen Herzen für die in der nachstehenden Tabelle aufgeführten Einzeldosen bestimmt.

b) Ergebnisse

| Verbindung | $ED_{50}$ | Wirkdauer (Dosis) |
|---|---|---|
| (+)-3,4-Dihydro-2,2-dimethyl-6-phenylsulfonyl-trans-4-(2-oxo-1-pyrrolidinyl)-2H-benzo[b]pyran-3-ol Beispiel 5a | 100 ng | 13 min (100 ng) |
| (±)-3,4-Dihydro-2,2-dimethyl-6-phenylsulfonyl-trans-4-(2-oxo-1-pyrrolidinyl)-2H-benzo[b]pyran-3-ol Beispiel 5 | 304 ng | 11 min (400 ng) |
| (±)-3,4-Dihydro-2,2-dimethyl-6-(2-methylphenylsulfonyl)-trans-4-(2-oxo-1-pyrrolidinyl)-2H-benzo[b]pyran-3-ol Beispiel 14 | 180 ng | 13 min (200 ng) |
| (±)-3,4-Dihydro-2,2-dimethyl-7-methoxy-6-phenylsulfonyl-trans-4-(2-oxo-1-pyrrolidinyl)-2H-benzo[b]pyran-3-ol Beispiel 3 | 670 ng | 14 min (500 ng) |

| Verbindung | ED50 | Wirkdauer (Dosis) |
|---|---|---|
| (+) -3,4-Dihydro-2,2-dimethyl-7-methoxy-6-phenylsulfonyl-trans-4-(2-oxo-1-pyrrolidinyl)-2H-benzo[b]pyran-3-ol Beispiel 3a | 370 ng | 11 min (500 ng) |
| (±)-3,4-Dihydro-2,2-dimethyl-trans-4-(2-oxo-1-pyrrolidinyl)-6-p-tolylsulfonyl-2H-benzo[b]-pyran-3-ol Beispiel 4 | 570 ng | 11 min (500 ng) |
| (±)-6-Cyano-3,4-dihydro-2,2-dimethyl-trans-4-(2-oxo-1-pyrrolidinyl)-2H-benzo[b]pyran-3-ol (Beispiel aus J. Med. Chem. 1986, 29, 2194-2201) | 590 ng | 6 min (500 ng) |
| (±)-2,2-Dimethyl-3,4-dihydro-7-methoxy-6-methyl-sulfonyl-trans-4-(2-oxo-1-pyrrolidinyl)-2H-benzo-[b]pyran-3-ol nach EP 173 848 | > 100 ng | - |

| Verbindung | $ED_{50}$ | Wirkdauer (Dosis) |
|---|---|---|
| (±)-2,2-Dimethyl-3,4-dihydro-6-methylsulfonyl-trans-4-(2-oxo-1-pyrrolidinyl)-2H-benzo[b]pyran-3-ol | 2770 ng | 5 min (2000 ng) |
| nach EP 173 848 | | |

**Patentansprüche**

**Patentansprüche für folgende Vertragsstaaten : AT, BE, CH, DE, FR, GB, IT, LI, LU, NL, SE**

**1.** 3,4-Dihydro-2H-benzo[b]pyrane der Formel I

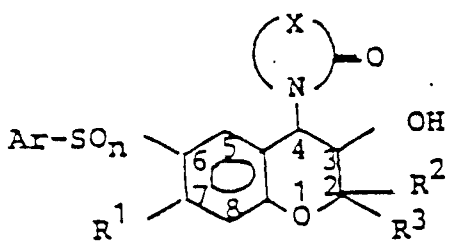

I

in welcher

- $R^1$ für H, OH, $(C_1\text{-}C_2)$-Alkoxy, $(C_1\text{-}C_2)$-Alkyl oder $NR^4R^5$ steht, wobei $R^4$ und $R^5$ gleich oder verschieden sind und für H, $(C_1\text{-}C_2)$Alkyl oder $(C_1\text{-}C_3)$-Alkylcarbonyl stehen,
- $R^2$,$R^3$ gleich oder verschieden sind und für Alkyl mit 1-4 C-Atomen stehen,
- Ar für Phenyl steht, das unsubstituiert oder durch 1 bis 3 gleiche oder verschiedene Reste $(C_1\text{-}C_2)$-Alkyl, $(C_1\text{-}C_2)$Alkoxy, Halogen, Trifluormethyl, CN, $NO_2$, CO-$(C_1\text{-}C_2)$Alkyl $SO_m$-$(C_1\text{-}C_2)$-Alkyl mit m = 1 oder 2 substituiert ist
- n für 1 oder 2 steht,
- X für eine Kette $(CH_2)_r$ steht, die durch ein Heteroatom O, S oder $NR^6$ unterbrochen sein kann, wobei $R^6$ H oder $(C_1\text{-}C_4)$-Alkyl bedeutet und r für die Zahlen 2, 3, 4 oder 5 steht,

wobei die Substituenten an C-Atomen 3 und 4 trans-Orientierung aufweisen.

**2.** Verbindung I nach Anspruch 1, dadurch gekennzeichnet, daß die Substituenten oder Indices folgende Bedeutung haben:

- $R^1$, $R^2$, $R^3$ und n wie in Anspruch 1 definiert,
- Ar Phenyl,
- X $(CH_2)_r$ mit r = 3 oder 4.

**3.** Verbindung I nach Anspruch 1, dadurch gekennzeichnet, daß die Substituenten oder Indices folgende Bedeutung haben:

- $R^1$, $R^2$ und $R^3$ wie in Anspruch 1 definiert,
- Ar Phenyl, das unsubstituiert oder wie in Anspruch 1 definiert substituiert ist,

X $(CH^2)_r$ mit r = 3 oder 4.

**4.** Verbindung I nach Anspruch 1, dadurch gekennzeichnet, daß die Substituenten oder Indices folgende Bedeutung haben:

| | |
|---|---|
| $R^1$ | H oder $(C_1\text{-}C_2)$ Alkoxy |
| $R^2$ und $R^3$ | wie in Anspruch 1 definiert. |
| Ar | Phenyl das unsubstituiert oder durch $(C_1\text{-}C_2)$-Alkyl, Cyano, $(C_1\text{-}C_2)$-Alkoxy oder Halogen einfach substituiert ist, |
| n | 2 |
| X | $(CH_2)_r$ mit r = 3 oder 4. |

**5.** Verbindung I nach Anspruch 1, dadurch gekennzeichnet, daß die Substituenten oder Indices folgende Bedeutung haben:

| | |
|---|---|
| $R^1$ | H oder $CH_3O$ |
| $R^2$ | = $R^3$ = $CH_3$ |
| Ar | = Phenyl |
| n | = 2 |
| X | = $(CH_2)_3$. |

**6.** Verfahren zum Herstellen von Verbindungen I nach Ansprüch 1, dadurch gekennzeichnet, daß man

a) Verbindungen der Formel II

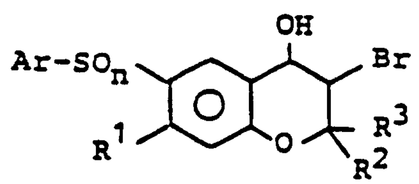

II,

in denen $R^1$ bis $R^3$ und $ArSO_n$ wie oben definiert sind umsetzt mit Lactamen der Formel III

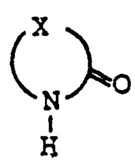

III,

b) Verbindungen der Formel IV

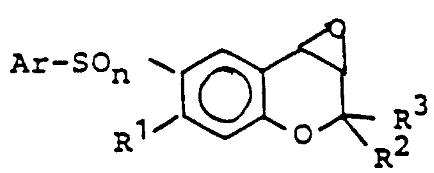

IV,

in denen $R^1$ bis $R^3$ und $ArSO_n$ wie oben definiert sind, umsetzt mit den Lactamen der III

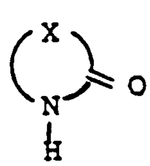

c) Verbindungen der Formel V

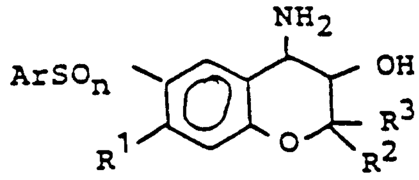

V,

in denen $R^1$ bis $R^3$ und $ArSO_n$ wie oben definiert sind, acyliert zu den Verbindungen VI

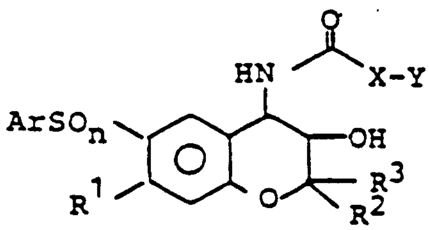

VI,

in denen Y eine Fluchtgruppe ist und $R^1$ bis $R^3$ und $ArSO_n$ wie oben definiert sind, und diese zu den Verbindungen I cyclisiert,
d) Verbindungen der Formel VII

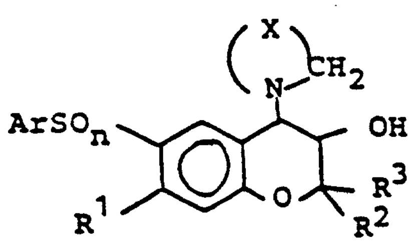

VII,

in denen $R^1$ bis $R^3$ und $ArSO_n$ wie oben definiert sind, zu den Verbindungen I oxidiert.

**7.** Verwendung einer Verbindung der Formel I nach Anspruch 1 als Mittel zur Behandlung der Hypertonie, der Angina pectoris oder der Herzinsuffizienz.

**8.** Verwendung einer Verbindung der Formel I nach Anspruch 1 zur Herstellung eines Arzneimittels gegen Hypertonie, der Angina pectoris oder der Herzinsuffizienz.

**9.** Arzneimittel zur Behandlung der Hypertonie, der Angina pectoris oder der Herzinsuffizienz, gekennzeichnet durch einenwirksamen Gehalt einer Verbindung I nach Anspruch 1.

**Patentansprüche für folgende Vertragsstaaten : ES, GR**

1. Verfahren zum Herstellen von Verbindungen I

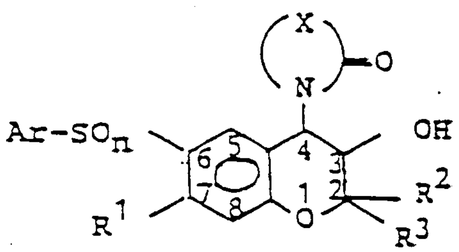

I

in welchen

- $R^1$ für H, OH, $(C_1\text{-}C_2)$-Alkoxy, $(C_1\text{-}C_2)$-Alkyl oder $NR^4R^5$ steht, wobei $R^4$ und $R^5$ gleich oder verschieden sind und für H, $(C_1\text{-}C_2)$Alkyl oder $(C_1\text{-}C_3)$-Alkylcarbonyl stehen,
- $R^2$,$R^3$ gleich oder verschieden sind und für Alkyl mit 1-4 C-Atomen stehen,
- Ar für Phenyl steht, das unsubstituiert oder durch 1 bis 3 gleiche oder verschiedene Reste $(C_1\text{-}C_2)$-Alkyl, $(C_1\text{-}C_2)$Alkoxy; Halogen, Trifluormethyl, CN, $NO_2$, CO-$(C_1\text{-}C_2)$Alkyl $SO_m$-$(C_1\text{-}C_2)$ mit m = 1 oder 2 substituiert ist
- n für 1 oder 2 steht,
- X für eine Kette $(CH_2)_r$ steht, die durch ein Heteroatom O, S oder $NR^6$ unterbrochen sein kann, wobei $R^6$ H oder $(C_1\text{-}C_4)$-Alkyl bedeutet und r für die Zahlen 2, 3, 4 oder 5 steht,

und in weichen die Substituenten an C-Atomen 3 und 4 trans-Orientierung aufweisen, dadurch gekennzeichnet, daß man

a) Verbindungen der Formel II

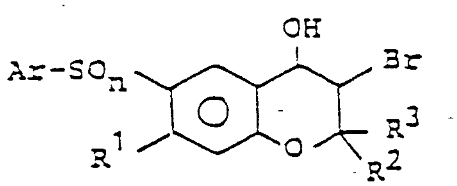

II,

in denen $R^1$ bis $R^3$ und $ArSO_n$ wie oben definiert sind umsetzt mit Lactamen der Formel III

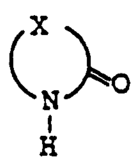

III,

b) Verbindungen der Formel IV

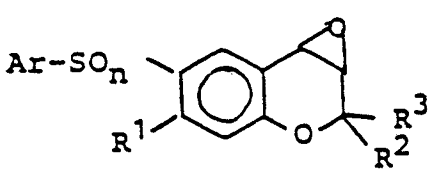

IV,

in denen $R^1$ bis $R^3$ und $ArSO_n$ wie oben definiert sind, umsetzt mit den Lactamen der III

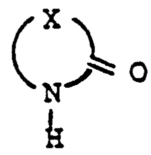

c) Verbindungen der Formel V

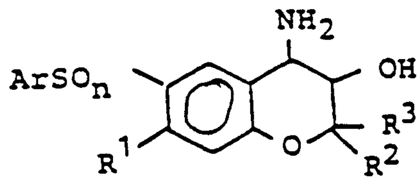

V,

in denen $R^1$ bis $R^3$ und $ArSO_n$ wie oben definiert sind, acyliert zu den Verbindungen VI

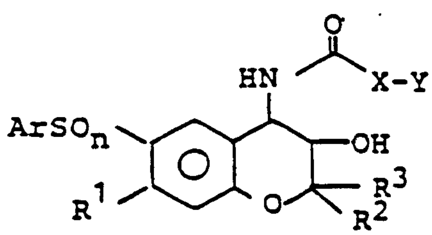

VI,

in denen Y eine Fluchtgruppe ist und $R^1$ bis $R^3$ und $ArSO_n$ wie oben definiert sind, und diese zu den Verbindungen I cyclisiert,

d) Verbindungen der Formel VII

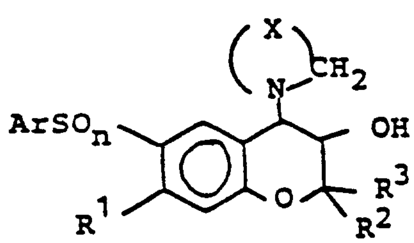

VII,

in denen $R^1$ bis $R^3$ und $ArSO_n$ wie oben definiert sind, zu den Verbindungen I oxidiert.

**2.** Verfahren nach Anspruch 1, dadurch gekennzeichnet, daß die Substituenten oder Indices folgende Bedeutung haben:

| | |
|---|---|
| $R^1$, $R^2$, $R^3$ und n | wie in Anspruch 1 definiert, |
| Ar | Phenyl, |
| X | $(CH_2)_r$ mit r = 3 oder 4. |

**3.** Verfahren nach Anspruch 1, dadurch gekennzeichnet daß die Substituenten oder Indices folgende Bedeutung haben:

| | |
|---|---|
| $R^1$, $R^2$ und $R^3$ | wie in Anspruch 1 definiert, |
| Ar | Phenyl, das unsubstituiert oder wie in Anspruch 1 definiert substituiert ist, |
| X | $(CH_2)_r$ mit r = 3 oder 4. |

**4.** Verfahren nach Anspruch 1, dadurch gekennzeichnet, daß die Substituenten oder Indices folgende Bedeutung haben:

| | |
|---|---|
| $R^1$ | H oder $(C_1-C_2)$ Alkoxy |
| $R^2$ und $R^3$ | wie in Anspruch 1 definiert |
| Ar | Phenyl, das unsubstituiert oder durch $(C_1-C_2)$-Alkyl Cyano, $(C_1-C_2)$Alkoxy oder Halogen einfach substituiert ist, |
| n | 2 |
| X | $(CH_2)_r$ mit r = 3 oder 4. |

**5.** Verfahren nach Anspruch 1, dadurch gekennzeichnet, daß die Substituenten oder Indices folgende Bedeutung haben:

| | |
|---|---|
| $R^1$ | H oder $CH_3O$ |
| $R^2$ | = $R^3$ = $CH_3$ |
| Ar | = Phenyl |
| n | = 2 |
| X | = $(CH_2)_3$. |

**Claims**

**Claims for the following Contracting States : AT, BE, CH, DE, FR, GB, IT, LI, LU, NL, SE**

**1.** A 3,4-dihydro-2H-benzo[b]pyran of the formula I

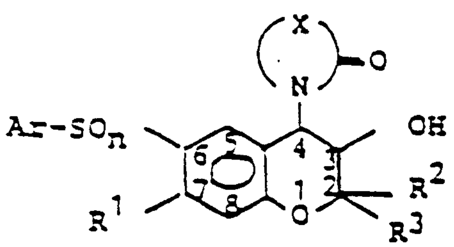

I

in which

| | |
|---|---|
| $R^1$ | is H, OH, $(C_1-C_2)$-alkoxy, $(C_1-C_2)$-alkyl or $NR^4R^5$, $R^4$ and $R^5$ being identical or different and representing H, $(C_1-C_2)$-alkyl or $(C_1-C_3)$-alkylcarbonyl, |
| $R^2$ and $R^3$ | are identical or different and are alkyl having 1-4 carbon atoms, |
| Ar | is phenyl which is unsubstituted or substituted by 1 to 3 identical or different radicals $(C_1-C_2)$-alkyl, $(C_1-C_2)$-alkoxy, halogen, trifluoromethyl, CN, $NO_2$, CO-$(C_1-C_2)$alkyl or $SO_m$-$(C_1-C_2)$-alkyl with m = 1 or 2, |
| n | is 1 or 2 and |
| X | is a $(CH_2)_r$ chain which can be interrupted by a heteroatom O, S or $NR^6$, $R^6$ being H or $(C_1-C_4)$-alkyl and r being the numbers 2, 3, 4 or 5, where the substituents on carbon atoms 3 and 4 have the trans orientation. |

**2.** A compound I as claimed in claim 1, wherein the substituents or indices have the following meaning:

| | |
|---|---|
| $R^1$, $R^2$, $R^3$ and n | as defined in claim 1, |
| Ar | = phenyl, and |
| X | = $(CH_2)_r$ with r = 3 or 4. |

**3.** A compound I as claimed in claim 1, wherein the substituents or indices have the following meaning:

| | |
|---|---|
| $R^1$, $R^2$ and $R^3$ | as defined in claim 1, |
| Ar = | phenyl which is unsubstituted or substituted as defined in claim 1, and |
| X | = $(CH_2)_r$ with r = 3 or 4. |

**4.** A compound I as claimed in claim 1, wherein the substituents or indices have the following meaning:

| | |
|---|---|
| $R^1$ | = H or $(C_1-C_2)$-alkoxy, |
| $R^2$ and $R^3$ | as defined in claim 1, |

Ar = phenyl which is unsubstituted or monosubstituted by $(C_1-C_2)$-alkyl, cyano, $(C_1-C_2)$-alkoxy or halogen,
n = 2 and
X = $(CH_2)_r$ with r = 3 or 4.

**5.** A compound I as claimed in claim 1, wherein the substituents or indices have the following meaning:
$R^1$ = H or $CH_3O$
$R^2$ = $R^3$ = $CH_3$
Ar = phenyl
n = 2
X = $(CH_2)_3$.

**6.** A process for the preparation of a compound I as claimed in claim 1, which comprises
a) reacting a compound of the formula II

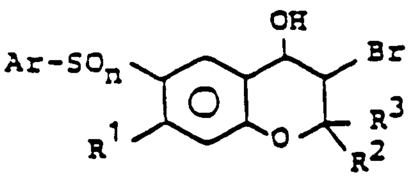

II,

in which $R^1$ to $R^3$ and $ArSO_n$ are as defined above, with a lactam of the formula III

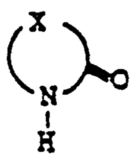

III,

b) reacting a compound of the formula IV

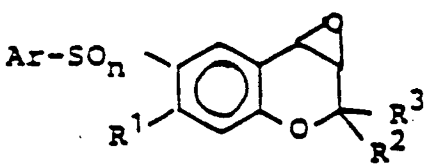

IV,

in which $R^1$ to $R^3$ and $ArSO_n$ are as defined above, with the lactam of the formula III

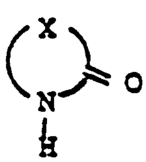

c) acylating a compound of the formula V

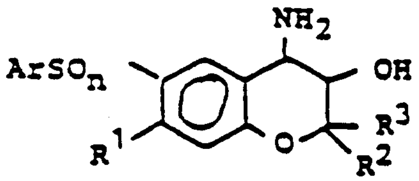

V,

in which $R^1$ to $R^3$ and $ArSO_n$ are as defined above, to give the compound VI

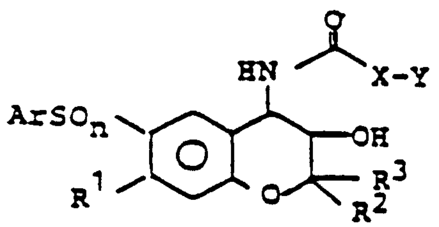

VI,

in which Y is a leaving group and $R^1$ to $R^3$ and $ArSO_n$ are as defined above, and cyclizing the latter to the compound I, or

d) oxidizing a compound of the formula VII

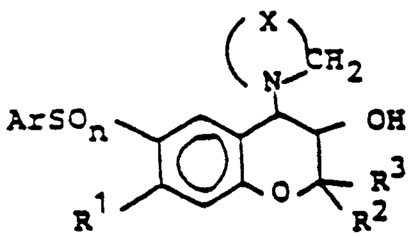

VII,

in which $R^1$ to $R^3$ and $ArSO_n$ are as defined above, to the compound I.

**7.** The use of a compound of the formula I as claimed in claim 1 as an agent for treating hypertension, angina pectoris or cardiac insufficiency.

**8.** The use of a compound of the formula I as claimed in claim 1 for the preparation of a medicament for treating hypertension, angina pectoris or cardiac insufficiency.

**9.** A medicament for treating hypertension, angina pectoris or cardiac insufficiency which has an effective content of a compound I as claimed in claim I.

**Claims for the following Contracting States : ES, GR**

**1.** A process for the preparation of a compound I

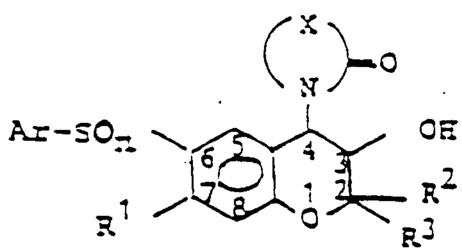

I

in which

| | |
|---|---|
| $R^1$ | is H, OH, $(C_1-C_2)$-alkoxy, $(C_1-C_2)$-alkyl or $NR^4R^5$, $R^4$ and $R^5$ being identical or different and representing H, $(C_1-C_2)$ -alkyl or $(C_1-C_3)$-alkylcarbonyl, |
| $R^2$ and $R^3$ | are identical or different and are alkyl having 1-4 carbon atoms, |
| Ar | is phenyl which is unsubstituted or substituted by 1 to 3 identical or different radicals $(C_1-C_2)$-alkyl, $(C_1-C_2)$-alkoxy, halogen, trifluoromethyl, CN, $NO_2$, CO-$(C_1-C_2)$-alkyl or $SO_m$-$(C_1-C_2)$-alkyl with |
| m | = 1 or 2, |
| n | is 1 or 2 and |
| X | is a $(CH_2)_r$ chain which can be interrupted by a heteroatom O, S or $NR^6$, $R^6$ being H or $(C_1-C_4)$-alkyl and r being the numbers 2, 3, 4 or 5 and in which the substituents on carbon atoms 3 and 4 have the trans orientation, |

which comprises

a) reacting a compound of the formula II

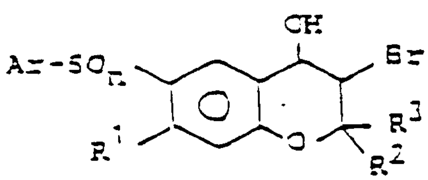

II,

in which $R^1$ to $R^3$ and $ArSO_n$ are as defined above, with a lactam of the formula III

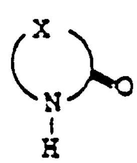

III,

b) reacting a compound of the formula IV

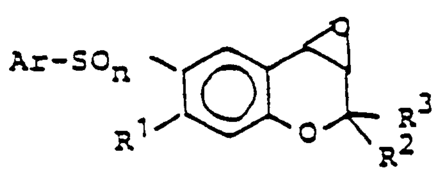

IV,

in which $R^1$ to $R^3$ and $ArSO_n$ are as defined above, with the lactam of the formula III

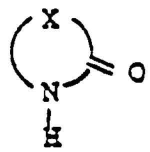

c) acylating a compound of the formula V

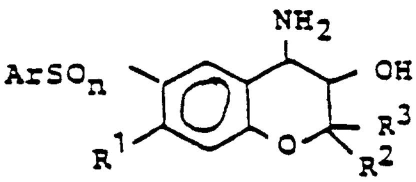

V,

in which $R^1$ to $R^3$ and $ArSO_n$ are as defined above, to give the compound VI

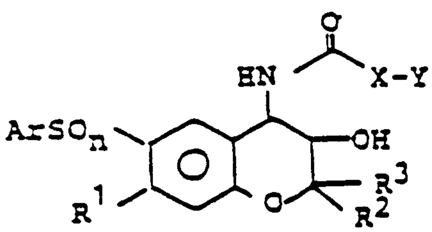

VI,

in which Y is a leaving group and $R^1$ to $R^3$ and $ArSO_n$ are as defined above, and cyclizing the latter to the compound I, or

d) oxidizing a compound of the formula VII

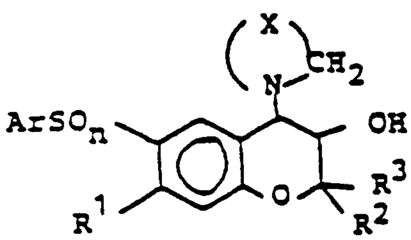

VII,

in which $R^1$ to $R^3$ and $ArSO_n$ are as defined above, to the compound I.

**2.** A process as claimed in claim 1, wherein the substituents or indices have the following meaning:

| | |
|---|---|
| $R^1$, $R^2$, $R^3$ and n | as defined in claim 1, |
| Ar | = phenyl, and |
| X | = $(CH_2)_r$ with r = 3 or 4. |

**3.** A process as claimed in claim 1, wherein the substituents or indices have the following meaning:

| | |
|---|---|
| $R^1$, $R^2$ and $R^3$ | as defined in claim 1, |
| Ar | = phenyl, which is unsubstituted or substituted as defined in claim 1, and |
| X | = $(CH_2)_r$ with r = 3 or 4. |

**4.** A process as claimed in claim 1, wherein the substituents or indices have the following meaning:

| | |
|---|---|
| $R^1$ | = H or $(C_1\text{-}C_2)$-alkoxy, |
| $R^2$ and $R^3$ | as defined in claim 1, |
| Ar | = phenyl which is unsubstituted or monosubstituted by $(C_1\text{-}C_2)$-alkyl, cyano, $(C_1\text{-}C_2)$- |

alkoxy or halogen,

n = 2 and

X = $(CH_2)_r$ with r = 3 or 4.

**5.** A process as claimed in claim 1, wherein the substituents or indices have the following meaning:

$R^1$ = H or $CH_3O$

$R^2$ = $R^3$ = $CH_3$

Ar = phenyl

n = 2

X = $(CH_2)_3$.

**Revendications**

**Revendications pour les Etats contractants suivants : AT, BE, CH, DE, FR, GB, IT, LI, LU, NL, SE**

**1.** 3,4-dihydro-2H-benzo[b]pyrannes de formule I

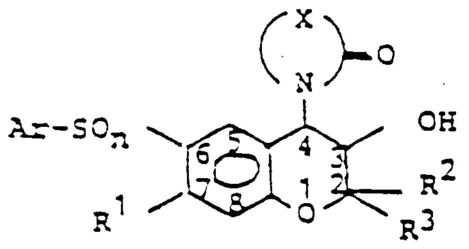

I

dans laquelle

$R^1$ représente H, OH, un groupe alcoxy en $C_1$-$C_2$, alkyle en $C_1$-$C_2$ ou $NR^4R^5$, $R^4$ et $R^5$ étant identiques ou différents et représentant H ou un groupe alkyle en $C_1$-$C_2$ ou alkyl ($C_1$-$C_3$)-carbonyle,

$R^2$, $R^3$ sont identiques ou différents et représentent un groupe alkyle ayant de 1 à 4 atomes de carbone,

Ar représente un groupe phényle qui est non substitué ou substitué par un 1 à 3 substituants, identiques ou différents, alkyle en $C_1$-$C_2$, alcoxy en $C_1$-$C_2$, halogène, trifluorométhyle, CN, $NO_2$, CO-alkyle($C_1$-$C_2$), $SO_m$-alkyle-($C_1$-$C_2$), m étant 1 ou 2,

n représente 1 ou 2,

X représente une chaîne $(CH_2)_r$ qui peut être interrompue par un hétéroatome O, S ou par $NR^6$, $R^6$ représentant H ou un groupe alkyle en $C_1$-$C_4$, et r représentant les nombres 2, 3, 4 ou 5,

les substituants sur les atomes de carbone 3 et 4 présentant l'orientation trans.

**2.** Composé I selon la revendication 1, caractérisé en ce que les substituants ou indices ont les significations suivantes:

$R^1$, $R^2$, $R^3$ et n sont tels que définis dans la revendication 1,

Ar est le groupe phényle,

X représente $(CH_2)_r$ avec r = 3 ou 4.

**3.** Composé I selon la revendication 1, caractérisé en ce que les substituants ou indices ont les significations suivantes:

$R^1$, $R^2$, $R^3$ sont tels que définis dans la revendication 1,

Ar représente un groupe phényle qui est non substitué ou substitué comme défini dans la revendication 1,

X représente $(CH_2)_r$ avec r = 3 ou 4.

**4.** Composé I selon la revendication 1, caractérisé en ce que les substituants ou indices ont les significations suivantes:

$R^1$ représente H ou un groupe alcoxy en $C_1$-$C_2$,

$R^2$ et $R^3$ sont tels que définis dans la revendication 1,

Ar représente un groupe phényle qui est non substitué ou substitué une fois par un atome d'halogène ou par un radical alkyle en $C_1$-$C_2$, cyano ou alcoxy en $C_1$-$C_2$,

n est 2,

X est $(CH_2)_r$ avec r = 3 ou 4.

**5.** Composé I selon la revendication 1, caractérisé en ce que les substituants ou indices ont les significations suivantes:

$R^1$ est H ou $CH_3O$,

$R^2$ = $R^3$ = $CH_3$,

Ar = phényle,

n = 2,

X = $(CH_2)_3$.

**6.** Procédé pour la préparation de composés I selon la revendication 1, caractérisé en ce que

a) on fait réagir des composés de formule II

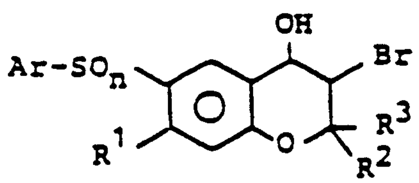

II

dans laquelle $R^1$ à $R^3$ et $ArSO_n$ sont tels que définis plus haut, avec des lactames de formule III

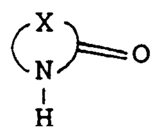

III

b) on fait réagir des composés de formule IV

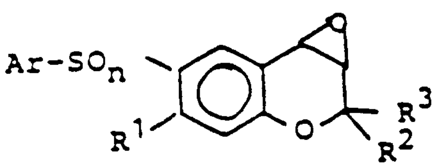

IV

dans laquelle $R^1$ et $R^3$ et $ArSO_n$ sont tels que définis plus haut, avec les lactames de formule III

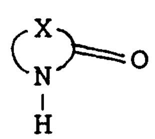

III

c) on soumet à une acylation des composés de formule V

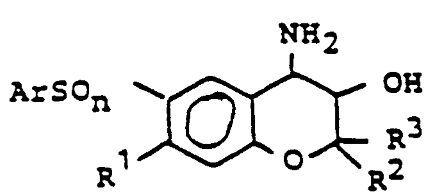

V

dans laquelle $R^1$ à $R^3$ et $ArSO_n$ sont tels que définis plus haut, pour aboutir aux composés VI

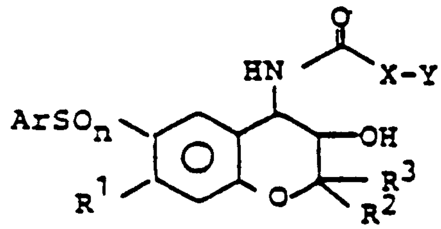

VI

dans lesquels Y est un groupe partant et $R^1$ à $R^3$ et $ArSO_n$ sont tels que définis plus haut, et on soumet ceux-ci à une cyclisation pour aboutir aux composés I,

d) on oxyde des composés de formule VII

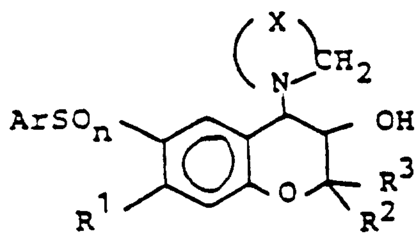

VII

dans laquelle $R^1$ à $R^3$ et $ArSO_n$ sont tels que définis plus haut, pour aboutir aux composés I.

**7.** Utilisation d'un composé de formule I selon la revendication 1, en tant qu'agent pour le traitement de l'hypertonie, de l'angine de poitrine ou de l'insuffisance cardiaque.

**8.** Utilisation d'un composé de formule I selon la revendication 1, pour la fabrication d'un médicament contre l'hypertonie, l'angine de poitrine ou l'insuffisance cardiaque.

**9.** Médicament pour le traitement de l'hypertonie, de l'angine de poitrine ou de l'insuffisance cardiaque, caractérisé par une teneur efficace en un composé I selon la revendication 1.

**Revendications pour les Etats contractants suivants : ES, GR**

**1.** Procédé pour la préparation de composés I

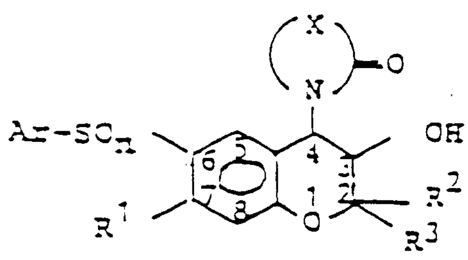

I

dans lesquels

- $R^1$ représente H, OH, un groupe alcoxy en $C_1$-$C_2$, alkyle en $C_1$-$C_2$ ou $NR^4R^5$, $R^4$ et $R^5$ étant identiques ou différents et représentant H ou un groupe alkyle en $C_1$-$C_2$ ou alkyl($C_1$-$C_3$)-carbonyle,
- $R^2$, $R^3$ sont identiques ou différents et représentent un groupe alkyle ayant de 1 à 4 atomes de carbone,
- Ar représente un groupe phényle qui est non substitué ou substitué par un 1 à 3 substituants, identiques ou différents, alkyle en $C_1$-$C_2$, alcoxy en $C_1$-$C_2$, halogène, trifluoromé-

thyle, CN, $NO_2$, CO-alkyle($C_1$-$C_2$),$SO_m$-alkyle($C_1$-$C_2$), m étant 1 ou 2,

n représente 1 ou 2,

X représente une chaîne $(CH_2)_r$ qui peut être interrompue par un hétéroatome O, S ou par $NR^6$, $R^6$ représentant H ou un groupe alkyle en $C_1$-$C_4$, et r représentant le nombre 2, 3, 4 ou 5,

et dans lesquels les substituants sur les atomes de carbone 3 et 4 présentent l'orientation trans, caractérisé en ce que

a) on fait réagir des composés de formule II

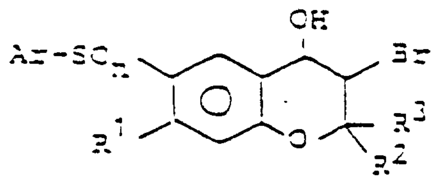

II

dans laquelle $R^1$ à $R^3$ et $ArSO_n$ sont tels que définis plus haut, avec des lactames de formule III

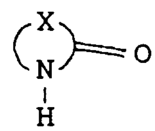

III

b) on fait réagir des composés de formule IV

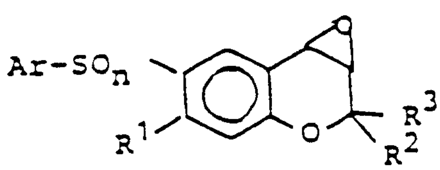

IV

dans laquelle $R^1$ et $R^3$ et $ArSO_n$ sont tels que définis plus haut, avec les lactames de formule III

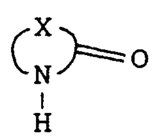

III

c) on soumet à une acylation des composés de formule V

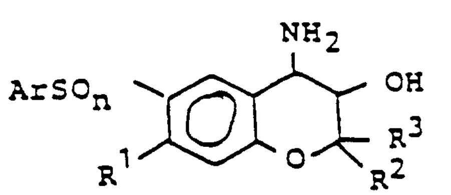

V

dans laquelle $R^1$ à $R^3$ et $ArSO_n$ sont tels que définis plus haut, pour aboutir aux composés VI

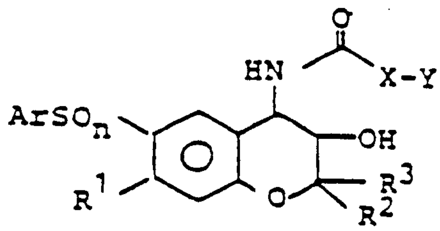

VI

dans lesquels Y est un groupe partant et $R^1$ à $R^3$ et $ArSO_n$ sont tels que définis plus haut, et on soumet ceux-ci à une cyclisation pour aboutir aux composés I,

d) on oxyde des composés de formule VII

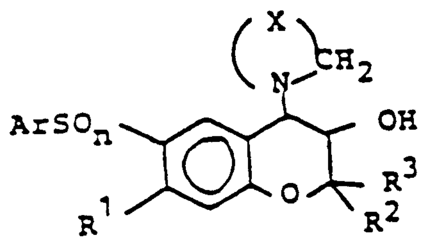

VII

dans laquelle $R^1$ à $R^3$ et $ArSO_n$ sont tels que définis plus haut, pour aboutir aux composés I.

**2.** Procédé selon la revendication 1, caractérisé en ce que les substituants ou indices ont les significations suivantes:

| | |
|---|---|
| $R^1$, $R^2$, $R^3$ et n | sont tels que définis dans la revendication 1, |
| Ar | est le groupe phényle, |
| X | représente $(CH_2)_r$ avec r = 3 ou 4. |

**3.** Procédé selon la revendication 1, caractérisé en ce que les substituants ou indices ont les significations suivantes:

| | |
|---|---|
| $R^1$, $R^2$, $R^3$ | sont tels que définis dans la revendication 1, |
| Ar | représente un groupe phényle qui est non substitué ou substitué comme défini dans la revendication 1, |
| X | représente $(CH_2)_r$ avec r = 3 ou 4. |

**4.** Procédé selon la revendication 1, caractérisé en ce que les substituants ou indices ont les significations suivantes:

| | |
|---|---|
| $R^1$ | représente H ou un groupe alcoxy en $C_1$-$C_2$, |
| $R^2$ et $R^3$ | sont tels que définis dans la revendication 1, |
| Ar | représente un groupe phényle qui est non substitué ou substitué une fois par un atome d'halogène ou par un radical alkyle en $C_1$-$C_2$, cyano ou alcoxy en $C_1$-$C_2$, |
| n | est 2, |
| X | est $(CH_2)_r$ avec r = 3 ou 4. |

**5.** Procédé selon la revendication 1, caractérisé en ce que les substituants ou indices ont les significations suivantes:

| | |
|---|---|
| $R^1$ | est H ou $CH_3O$, |
| $R^2$ | = $R^3$ = $CH_3$, |
| Ar | = phényle, |
| n | = 2, |
| X | = $(CH_2)_3$. |